# EUROPÄISCHE PATENTSCHRIFT

(11) **EP 4 434 467 B1**
(45) Veröffentlichungstag und Bekanntmachung des Hinweises auf die Patenterteilung: **04.06.2025**
(21) Anmeldenummer: 23190831.0
(22) Anmeldetag: 10.08.2023
(51) Int. Cl.: A61B 6/03, A61B 6/00, A61B 6/50

(54) **VERFAHREN UND STEUEREINRICHTUNG ZUR STEUERUNG EINES COMPUTERTOMOGRAPHIE-SYSTEMS**
METHOD AND CONTROL DEVICE FOR CONTROLLING A COMPUTER TOMOGRAPHY SYSTEM
PROCÉDÉ ET DISPOSITIF DE COMMANDE POUR COMMANDER UN SYSTÈME DE TOMOGRAPHIE ASSISTÉE PAR ORDINATEUR

(43) Veröffentlichungstag der Anmeldung: 25.09.2024
(73) Patentinhaber: Siemens Healthineers AG, 91301 Forchheim (DE)
(72) Erfinder: Lichy, Matthias, 90489 Nürnberg (DE); Lindner, Claudia, 90475 Nürnberg (DE); Hofmann, Bernd, 91058 Erlangen (DE); Schmidt, Bernhard, 90766 Fürth (DE)
(74) Vertreter: Siemens Healthineers Patent Attorneys

(56) Entgegenhaltungen:
- EP-A2- 1 764 040
- WO-A1-2020/142404
- DE-A1- 102011 055 616

## Beschreibung

Die Erfindung betrifft ein Verfahren und eine Steuereinrichtung zur Steuerung eines Computertomographie-Systems sowie ein Computertomographie-System ("CT-System").

Bei CT-Untersuchungen von lokal begrenzter Anatomie ("CT": Computertomographie), **z.B.** der Brust, wird mit der herkömmlichen Aufnahmetechnik und Bildrekonstruktion eine für die gewünschte dargestellte Anatomie unzureichende Anzahl von Projektionswinkeln erfasst. Im konkreten Fall würden für die Darstellung der Brust, Axilla und vorderen Thoraxwand Projektionen durch die Wirbelsäule verwendet, die eine hohe Dosis aufweisen und darüber hinaus Artefakt-belastet sein können. Sie können z.B. Aufhärtungsartefakte aufweisen.

Darüber hinaus ist insbesondere bei der (weiblichen) Brustbildgebung die Verwendung dedizierter Lagerungshilfen (kompressionsfreie "hängende" Darstellung des Parenchyms) notwendig. Diese verursachen jedoch eine suboptimale Lagerung der Patientin für eine konventionelle Datenakquisition und Datenrekonstruktion (off-center Lagerung).

Auch für eine Reihe von Untersuchungen ohne dedizierte Lagerungshilfe tritt eine Reduktion der Bildqualität auf, z.B. laterale Lagerung bei Leberintervention.

Die für die Kontrolle von Interventionen und Strahlentherapielagerung durchgeführten Schnittbildtechniken (z.B. Flatpanel-CT) zeigen eine gleiche Grundproblematik und unterscheiden sich nur in der Thematik der ausgeprägten Kegelstrahlprojektion im Vergleich zur konventionellen CT-Technik. In Bezug auf die beispielhaft genannte CT-basierte Brustbildgebung existiert ein dediziertes Brust-CT. Bei der dabei angewandten Technik ist die Darstellung der Thoraxwand und der Axilla aufgrund der technisch-physikalischen Gegebenheiten des Systems nicht möglich. Ebenso ist eine zeitgleiche Darstellung beider Brüste nicht möglich, welches potentiell für dynamische Untersuchungen (z.B. Kontrastmitteldynamik) negative Auswirkungen auf die klinische Verwendung hat. Darüber hinaus ist die gleichzeitige Bildgebung und Zugänglichkeit der Brust für Interventionen wie sie in der MRT realisiert ist, mit einem solchen System aktuell nicht möglich.

Zur Zeit werde zum Entgegenwirken des Problems lediglich Lagerungshilfen auf individueller Basis eingesetzt. Im Bereich der CT-basierten Brustbildgebung gibt es in Analogie zur MRT individuell angefertigte Lagerungshilfen für die Bildgebung bzw. bei dedizierten Brustscannern entsprechende baulichen Maßnahmen zur Platzierung der Brüste.

Es ist eine Aufgabe der vorliegenden Erfindung, ein alternatives, komfortableres Verfahren und eine entsprechende Vorrichtung zur Steuerung eines Computertomographiesystems anzugeben, mit dem die oben beschriebenen Nachteile vermieden werden.

Diese Aufgabe wird durch ein Verfahren gemäß Patentanspruch 1, eine Steuereinrichtung gemäß Patentanspruch 10 und ein Computertomographie-System gemäß Patentanspruch 12 gelöst.

Das erfindungsgemäßen Verfahren dient zur Steuerung eines Computertomographie-Systems. Es erlaubt eine Verbesserung der Bildqualität und bietet zudem eine Dosisreduktion für den Patienten, insbesondere bei der Verwendung hochauflösender CT-Techniken wie z.B. der Photonen zählenden Technologie. Gerade bei der Aufnahme von anatomischen Regionen in welchen eine hohe Auflösung bei gleichzeitig geringem Kontrastverhalten für eine diagnostische Aussage von Vorteil sind, bietet das Verfahren die Kontrolle von Bildartefakten bei gleichzeitig vergleichsweise niedriger Strahlendosis.

Das Verfahren umfasst die folgenden Schritte:
- Festlegen eines Untersuchungsbereichs eines Untersuchungsobjekts,
- Festlegen einer Anzahl von Störstrukturen, wobei eine Störstruktur eine Struktur ist, die innerhalb des Strahlengangs des Computertomographie-Systems liegt und Röntgenstrahlung mit einer Stärke außerhalb eines Toleranzbereichs reflektiert oder absorbiert,
- Berechnen der Strahlengänge der Röntgenstrahlen einer CT-Untersuchung des Untersuchungsbereichs und Ermitteln von Stör-Winkelbereichen einer Gantry des Computertomographie-Systems, bei denen Strahlengänge durch eine Störstruktur und den Untersuchungsbereich führen,
- Durchführen einer CT-Aufnahme während die Gantry rotiert, und Aufnehmen von CT-Daten,
- Rekonstruieren eines Bilddatensatzes aus den CT-Daten unter Auslassung von ermittelten Stör-Winkelbereichen,
- Ausgeben des Bilddatensatzes.

Das Untersuchungsobjekt kann theoretisch ein beliebiger Körper sein und der Untersuchungsbereich ein Teil dieses Körpers oder der gesamte Körper. Bevorzugt ist das Untersuchungsobjekt ein Mensch und der Untersuchungsbereich ein Organ oder ein Körperbereich. Eine Festlegung des Untersuchungsbereichs erfolgt typischerweise vor der Untersuchung.

Ebenfalls im Vorfeld der Untersuchung wird eine Anzahl von Störstrukturen festgelegt. Dies kann eine einzige sein, aber auch mehrere. Eine Störstruktur liegt dabei innerhalb des Strahlengangs des Computertomographie-Systems, wird also von Röntgenstrahlung getroffen und reflektiert oder absorbiert diese. Es werden aber nicht beliebige Strukturen als Störstrukturen angesehen, sondern nur diejenigen, deren Reflexion bzw. Absorption eine Stärke außerhalb eines Toleranzbereichs aufweisen, z.B. über oder unter einem vorgegebenen Grenzwert liegen. Der Toleranzbereich oder entsprechende Materialien und Abmessungen für Strukturen werden dabei vorher festgelegt. Bei einem Menschen kann z.B. Knochenmaterial störend sein und größere Knochen wie insbesondere die Wirbelsäule, können als Störstrukturen angesehen werden. Es könnten aber auch Teile der Patientenliege oder Lagerungshilfen als Störstrukturen angesehen werden. Im Grunde ist einem Mediziner bekannt, welche Strukturen einen störenden Einfluss auf die Bilder haben. Diese Strukturen können dann als "Störstrukturen" für das Verfahren definiert werden. Bevorzugte Störstrukturen haben eine bekannte Position im Körper oder im Bereich der Untersuchung und sind Bildbereiche mit hohen Hounsfield Units. Es kann aber auch Luft eine Störstruktur darstellen. In diesem Falle sind als Störbereiche Bildbereiche mit den niedrigsten Hounsfield Units bevorzugt.

Wenn die Störstrukturen bekannt sind, ist auch im Grunde ihre Lage bekannt. Bei einem Menschen ist z.B. der Knochenbau bekannt und die Lage der Wirbelsäule als beispielhafte Störstruktur kann einfach angegeben werden auch wenn man in den Menschen (zu diesem Zeitpunkt noch) nicht hereinschauen kann.

Mit der bekannten Lage der Störstruktur und der festgelegten Lage des Untersuchungsbereichs können nun Strahlengänge der Röntgenstrahlen einer CT-Untersuchung berechnet werden. Wohlgemerkt ist dabei die Röntgenquelle noch ausgeschaltet. Es werden lediglich die theoretischen Verläufe bestimmt. Dabei ist bevorzugt, dass der Patient (bzw. das Untersuchungsobjekt) so positioniert wird, das die Anzahl der Störstrukturen (deutlich) exzentrisch des Isocenters liegen.

Mit den berechneten Strahlverläufen wird dann ermittelt, ob diese durch eine Störstruktur und den Untersuchungsbereich führen. Die Winkel unter denen die Röntgenquelle Röntgenstrahlen emittieren würde, die durch eine Störstruktur und den Untersuchungsbereich führen, wird als "Stör-Winkel" bezeichnet und der Bereich der Stör-Winkel als "Stör-Winkelbereich". Die Stör-Winkelbereiche sind also diejenigen Winkelbereiche, die die rotierende Gantry annehmen kann, in denen Röntgenstrahlen emittiert würden, die durch eine Störstruktur und den Untersuchungsbereich führen. Es kann dabei bevorzugt toleriert werden, wenn ein Röntgenstrahl erst nach Passieren des Untersuchungsbereichs durch die Störstruktur verlaufen würde. Diese Winkelbereiche können also bevorzugt nicht als Stör-Winkelbereiche angesehen werden.

Nachdem die Stör-Winkelbereiche festgelegt sind, kann dann eine CT-Aufnahme mit einer rotierenden Gantry durchgeführt werden und dabei CT-Daten (also im Grunde Bilder) aufgenommen werden.

Aus diesen CT-Daten wird dann ein Bilddatensatz rekonstruiert. Dabei werden ermittelte Stör-Winkelbereiche ausgelassen. Wohlgemerkt müssen dabei nicht zwingend alle Stör-Winkelbereiche ausgelassen werden. Beispielsweise kann beim Vorliegen von zwei getrennten Untersuchungsbereichen ein erster Stör-Winkelbereich für die Rekonstruktion eines ersten Bilddatensatzes des ersten Untersuchungsbereichs ausgelassen werden und ein zweiter Stör-Winkelbereich für die Rekonstruktion eines zweiten Bilddatensatzes des zweiten Untersuchungsbereichs.

Eine Reduktion der Dosis kann dadurch erreicht werden, dass bei der Aufnahme eine Röntgenquelle in den Stör-Winkelbereichen abgeschaltet wird.

Der rekonstruierte Bilddatensatz wird dann ausgegeben. Er kann z.B. für eine direkte Befundung dargestellt werden oder für eine spätere Befundung abgespeichert werden.

Durch das Verfahren werden also im Grunde Areale aus den möglichen CT-Daten herausgeschnitten, die das Bild verschlechtern würden. Hierzu ist zu beachten, dass für eine Rekonstruktion typischerweise ein Aufnahmewinkel von etwas mehr als 180° benötigt wird. Es ist also von Vorteil, wenn bei gegenüberliegenden Stör-Winkelbereichen derjenige Winkelbereich nicht als Stör-Winkelbereich angesehen wird, bei dem sie Störungen geringer sind. Alternativ oder zusätzlich können auch CT-Daten für Stör-Winkelbereiche simuliert oder (z.B. mit einer entsprechend trainierten KI) berechnet werden und für die Rekonstruktion verwendet werden.

Mit den Stör-Winkelbereichen können die notwendigen Projektionen für den Untersuchungsbereich unter Vermeidung von Bildqualität einschränkenden und somit dosisineffizienten Projektionen festgelegt werden. Konkret werden z.B. Strahlengängen durch die knöcherne Wirbelsäule vermieden. Dies kann sowohl für sequentielle als auch für spiralförmige Aufnahmen erfolgen.

Die erfindungsgemäße Steuereinrichtung zur Steuerung eines Computertomographie-Systems ist bevorzugt zur Ausführung des erfindungsgemäßen Verfahrens ausgelegt. Sie umfasst die folgenden Komponenten:
- eine Untersuchungseinheit ausgelegt zum Festlegen eines Untersuchungsbereichs eines Untersuchungsobjekts,
- eine Störstruktur-Einheit ausgelegt zum Festlegen einer Anzahl von Störstrukturen, wobei eine Störstruktur eine Struktur ist, die innerhalb des Strahlengangs des Computertomographie-Systems liegt und Röntgenstrahlung mit einer Stärke außerhalb eines Toleranzbereichs reflektiert oder absorbiert,
- eine Berechnungseinheit ausgelegt zum Berechnen der Strahlengänge der Röntgenstrahlen einer CT-Untersuchung des Untersuchungsbereichs und Ermitteln von Stör-Winkelbereichen einer Gantry des Computertomographie-Systems, bei denen Strahlengänge durch eine Störstruktur und den Untersuchungsbereich führen,
- eine Datenschnittstelle ausgelegt zur Ausgabe von Steuerbefehlen zum Durchführen einer CT-Aufnahme während die Gantry rotiert, und zum Empfang von CT-Daten,
- eine Rekonstruktionseinheit ausgelegt zum Rekonstruieren eines Bilddatensatzes aus den CT-Daten unter Auslassung von ermittelten Stör-Winkelbereichen,
- eine Datenschnittstelle ausgelegt zum Ausgeben des Bilddatensatzes.

Die Funktion der Komponenten wurde vorangehend im Rahmen des erfindungsgemäßen Verfahrens ausführlich beschrieben.

Ein erfindungsgemäßes Computertomographiesystem umfasst eine erfindungsgemäße Steuereinrichtung oder ist alsternativ oder zusätzlich zur Ausführung des erfindungsgemäßen Verfahrens ausgelegt. Bevorzugt umfasst das CT-System einen Flat-panel-Detektor.

Ein Großteil der zuvor genannten Komponenten der Steuereinrichtung, können ganz oder teilweise in Form von Softwaremodulen in einem Prozessor eines entsprechenden Rechensystems realisiert werden. Eine weitgehend softwaremäßige Realisierung hat den Vorteil, dass auch schon bisher verwendete Rechensysteme auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem ladbar ist, mit Programmabschnitten, um die Schritte des erfindungsgemäßen Verfahrens, zumindest die durch einen Computer ausführbaren Schritte auszuführen, wenn das Programm in dem Rechensystem ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Es sei angemerkt, dass eine Durchführung einer CT-Aufnahme der Übersendung von entsprechenden Steuerbefehlen und dem Empfang von CT-Daten entspricht. Das Verfahren kann insbesondere computerimplementiert sein.

Zum Transport zum Rechensystem bzw. zur Steuereinrichtung und/oder zur Speicherung an oder in dem Rechensystem bzw. der Steuereinrichtung kann ein computerlesbares Medium, z.B. ein Memorystick, eine Festplatte oder ein sonstiger transportabler oder fest eingebauter Datenträger dienen, auf welchem die von einem Rechensystem einlesbaren und ausführbaren Programmabschnitte des Computerprogramms gespeichert sind. Das Rechensystem kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Gemäß einem bevorzugten Verfahren werden die CT-Daten unter Auslassung von ermittelten Stör-Winkelbereichen aufgenommen. Dabei ist bevorzugt, dass eine zur Aufnahme verwendete Röntgenquelle in Stör-Winkelbereichen abgeschaltet wird oder zumindest ihre Strahlungsleistung verringert wird. Dadurch wird die Gesamtdosis verringert. Bevorzugt erfolgt eine Dosismodulation mit einer Änderung von Strahlparametern, z.B. kV-Setting oder Referenz-mAs.

Möchte man bei gleichbleibender Gesamtdosis die Bilder verbessern, dann werden bevorzugt Strahlparameter, insbesondere die Beschleunigungsspannung und/oder die Stromstärke der Röntgenquelle, für die gesamte Aufnahme basierend auf einem gesamten Winkelbereich von 360° abzüglich der Stör-Winkelbereiche bestimmt.

Gemäß einem bevorzugten Verfahren wird als Stör-Winkelbereich ein Winkelbereich angesehen, bei dem Strahlen einer Röntgenquelle der Gantry zuerst durch eine Störstruktur und danach durch den Untersuchungsbereich führen. Da der Strahl zumeist kegelförmig ist, haben diese Stör-Winkelbereiche auf die Aufnahmen einen gravierenderen Einfluss als die jeweils gegenüberliegenden Winkelbereiche, bei denen die Röntgenstrahlung zuerst den Untersuchungsbereich passierten und erst danach auf die Störstruktur treffen. Letztere Winkelbereiche können dann als Winkelbereiche angesehen werden, die für eine Rekonstruktion (und Aufnahme) verwendet werden sollen.

Gemäß einem bevorzugten Verfahren basiert das Festlegen einer Störstruktur auf einem Topogramm des Untersuchungsobjekts, einer Kameraaufnahme des Untersuchungsobjekts oder einem Modell des Untersuchungsobjekts.

Das Untersuchungsobjekt ist bevorzugt ein menschlicher oder tierischer Körper und eine Störstruktur ein Knochen, eine Lagerungshilfe oder ein Teil der Gantry.

Gemäß einem bevorzugten Verfahren wird das Untersuchungsobjekt mittels einer Lagerungshilfe gelagert. Das Festlegen einer Störstruktur basiert dann bevorzugt auf einer Position und einer Form der Lagerungshilfe. In diesem Fall ist also die Lagerungshilfe eine Störstruktur. Es ist dabei bevorzugt, dass das Isozentrum des Untersuchungsbereichs nach Lagerung des Untersuchungsobjekts mittels der Lagerungshilfe festgelegt wird. Diese Festlegung erfolgt bevorzugt automatisiert mittels einer Bilderkennung oder mittels einer Kommunikation zwischen Lagerungshilfe und Computertomographie-System.

Gemäß einem bevorzugten Verfahren wird das Untersuchungsobjekt mittels einer Fixation fixiert. Das Festlegen einer Störstruktur basiert dann bevorzugt auf einer Position und einer Form der Fixation. In diesem Fall ist also die Fixation, z.B. eine Schraube, eine Platte oder ein sonstiger starrer Körper, eine Störstruktur. Diese Festlegung erfolgt auch hier bevorzugt automatisiert mittels einer Bilderkennung oder mittels einer Kommunikation zwischen Fixation und Computertomographie-System.

In der Praxis kann nach einer Einbringung einer dedizierten Lagerungshilfe in den Scanner über eine manuelle Eingabe, Bilderkennung oder durch die direkte Kommunikation zwischen Lagerungshilfe und Scanner eine Kalibrierung des neuen Isocenters in Bezug auf die zu interessierende Anatomie erfolgen. Im Fall der Brustdarstellung könnte dem CT-Scanner eine vordefinierte Zielregion mitgeteilt werden, z.B. das Volumen des Brustkörbchens, oder die Lage oder Ausdehnung der Brust. Entsprechendes gilt für eine Fixation.

Eine bevorzugte Steuereinrichtung umfasst diesbezüglich eine Positionseinheit ausgelegt zum Ermitteln einer Position einer Lagerungshilfe und/oder Fixation. Es ist dabei bevorzugt, dass die Positionseinheit dazu ausgelegt ist, basierend auf Bildern die Position abzuschätzen und/oder Informationen der Lagerungshilfe zu empfangen und auszuwerten.

Gemäß einem bevorzugten Verfahren wird im Rahmen der Ermittlung der Stör-Winkelbereiche überprüft, ob unter Berücksichtigung des gesamten Winkelbereichs von 360° abzüglich der Stör-Winkelbereiche der verbleibende Winkelbereich überlagert mit dem verbleibenden Winkelbereich gedreht um 180° einen durchgehenden Winkelbereich von 360° aufweist. Es wird also im Grunde überprüft, ob für eine Rekonstruktion mindestens 180° zur Verfügung stehen. Wohlgemerkt muss diese Überprüfung nicht zwangsläufig durch das Verdrehen und Überlagern einer Kopie des verbleibenden Winkelbereichs erfolgen. Wenn der verbleibende Winkelbereich jedoch eine komplizierte Struktur hat, dann wäre dieses Überprüfungsverfahren vorteilhaft. Wenn bei einer solchen Überlagerung Löcher vorhanden sein sollten, dann stehen für eine Rekonstruktion keine 180° zur Verfügung, es fehlen also in diesem Fall Informationen.

Es ist dabei bevorzugt, dass in dem Fall, dass der verbleibende Winkelbereich kleiner als 360° ist, ein fehlender Winkelbereich vor der Rekonstruktion des Bilddatensatzes den CT-Daten simulierte Daten hinzugefügt werden, die Aufnahmen im fehlenden Winkelbereich simulieren. Alternativ oder zusätzlich kann ein fehlender Winkelbereich aus er Anzahl der Stör-Winkelbereiche entfernt werden, da die Stör-Winkelbereiche ja schon vor einer Aufnahme bekannt sind. Dazu ist bevorzugt, dass ein Grad der Störung im fehlenden Winkelbereich und einem dazu 180° gedrehten Winkelbereich ermittelt wird und derjenige Winkelbereich aus den Stör-Winkelbereichen entfernt wird, der den geringeren Grad der Störung aufweist. Dies ist bevorzugt derjenige Winkelbereich, bei dem der Röntgenstrahl zuerst den Untersuchungsbereich passiert und erst dann auf die Störstruktur trifft.

Gemäß einem bevorzugten Verfahren werden zwei oder mehr Untersuchungsbereiche des Untersuchungsobjekts festgelegt. Dabei werden Stör-Winkelbereiche für beide Untersuchungsbereiche bevorzugt separat ermittelt. Dabei wird ein Stör-Winkelbereich einem Untersuchungsbereich dann zugeordnet, wenn Strahlengänge durch eine Störstruktur und den betreffenden Untersuchungsbereich führen. Für jeden Untersuchungsbereich wird dann ein Bilddatensatz separat unter Auslassung von ermittelten Stör-Winkelbereichen des betreffenden Untersuchungsbereichs rekonstruiert. Wird also z.B. eine Störstruktur festgelegt und gibt es einen ersten Stör-Winkelbereich für den ersten Untersuchungsbereich und einen zweiten Stör-Winkelbereich für den zweiten Untersuchungsbereich, dann wird der Bilddatensatz für den ersten Untersuchungsbereich unter Auslassung des ersten Stör-Winkelbereichs rekonstruiert und der Bilddatensatz für den zweiten Untersuchungsbereich unter Auslassung des zweiten Stör-Winkelbereichs rekonstruiert.

Bevorzugt wird ermittelt, welche den Untersuchungsbereichen zugeordneten Stör-Winkelbereiche identisch sind. Dann können die CT-Daten unter Auslassung der identischen Stör-Winkelbereiche aufgenommen werden. Wenn mehrere Untersuchungsbereiche vorliegen können die jeweiligen Stör-Winkelbereiche disjunkt sein. Die Röntgenquelle sollte dann in einem Stör-Winkelbereich nicht ausgeschaltet werden, weil dann für einen Untersuchungsbereich nicht genügend Daten für die Rekonstruktion vorhanden sein könnten. Gibt es aber einen gemeinsamen Stör-Winkelbereich, dann kann dort die Röntgenquelle ausgeschaltet werden, um die Gesamtdosis zu verringern. Es wird also bevorzugt eine zur Aufnahme verwendete Röntgenquelle in den identischen Stör-Winkelbereichen abgeschaltet oder zumindest ihre Strahlungsleistung verringert.

Dadurch kann eine Optimierung für multiple zu untersuchende Areale erfolgen. Beispielsweise würde eine Kontrastmitteldynamik beider Brüste für die Bildakquisition eine andere Projektionsanzahl benötigen als für die einzelne Darstellung einer zu biopsierenden Brustläsion. Unabhängig hiervon erfolgt die Rekonstruktion bevorzugt basierend auf den optimalen Projektionen für die einzelnen Zielvolumina separat.

Es ist auch bevorzugt, dass ein Bilddatensatz aus zwei unterschiedlichen Projektionssets rekonstruiert wird. Im Beispiel der Kontrastmitteldynamik könnte die Akquisition auf die Notwendigkeit der simultanen Erfassung beider Brüste optimiert werden. Die Bildrekonstruktion kann bevorzugt auf individuellen optimierten Projektionen basieren, welche dann in einem Bild wieder zusammengefasst werden können. Für diese unterschiedlichen Projektionssets können zwei unterschiedliche Stör-Winkelbereiche existieren, so dass auch hierfür die Merkmale für zwei Untersuchungsbereiche angewandt werden können.

Gemäß einem bevorzugten Verfahren wird bei der Rekonstruktion eines Bilddatensatzes eine Kombination einer Data-Sparsity-Rekonstruktionstechnik mit einem iterativen Lösungsansatz verwendet. Dabei ist eine Zumischung von Projektionen zur Darstellung von Strukturen bevorzugt, insbesondere von Biopsienadeln ohne Notwendigkeit der vollen Datenakquisition wie sie für die Feindarstellung des Brustgewebes notwendig sind. Solche Verfahren sind im Stand der Technik bekannt und werden beispielsweise in der CT zur Berechnung von Daten außerhalb der durch die Detektoren komplett erfassten Areale verwendet (extentend field of view). Für das erfindungsgemäße Verfahren sind diese Techniken besonders vorteilhaft, wenn die Röntgenquelle in Stör-Winkelbereichen abgeschaltet wird, also von dort keine CT-Daten aufgenommen werden.

Gemäß einem bevorzugten Verfahren ist der Untersuchungsbereich die Brust oder die Lunge. Eine bevorzugte StörStruktur ist dabei die Wirbelsäule.

Es sei angemerkt, dass eine Störstruktur nicht zwingend besonders viel Strahlung absorbieren oder Reflektieren muss, sondern je nach Art einer Untersuchung kann eine Störstruktur auch besonders wenig Strahlung reflektieren oder absorbieren (also weniger als ein vorgegebener Grenzwert). Beispielsweise werden bei einer Biopsie eines Knochenprozesses im Beckenkamm Projektionen bevorzugt, die viel Information über den Knochen unter Aussparung von Projektionen mit hoher Weichteilinformation enthalten. Hier könnten Störstrukturen mit A priori Wissen und/oder Topogrammen festgelegt werden.

Bevorzugt ist auch eine Kombination aus konventioneller Dosismodulation mit einer selektiven Projektionsselektion (Auslassung von Stör-Winkelbereichen) für die dedizierte Rekonstruktion (z.B. gezoomte Wirbelsäule). Dadurch kann eine Verbesserung der Bildqualität bei erhalten gebliebener Strahleneffizienz erreicht werden. Das Verfahren könnte auch ein Ersatz bzw. als eine Ergänzung zu bisherigen Ansätzen der Bildqualitätsverbesserung herangezogen werden. Beispielsweise könnte, basierend auf der Information eines Topogramms ein Fremdkörper im Untersuchungsobjekt erkannt und dieser als Störstruktur festgelegt werden. Dann können, z.B. zur Vermeidung von Artefakten, z.B. Metallartefakten, Stör-Winkelbereiche für diesen Fremdkörper festgelegt werden. Beispielsweise könnte bei einer Lungendarstellung eine Aussparung der Projektionen basierend auf einem Fremdkörper, der in die Lunge eingedrungen ist, erfolgen. Dies stellt einen bedeutenden Unterschied zur iterativen Artefakt-Reduktion dar, da in der vorgeschlagenen Methode ein bereits genaues Wissen mit dem eingebrachten Fremdkörper vorliegt und prospektiv Projektionen aussortiert werden können.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 grob schematische Darstellung eines CT-Systems mit einem Ausführungsbeispiel einer erfindungsgemäßen Steuereinrichtung zur Durchführung des Verfahrens.
Figur 2 eine Störstruktur und einen Stör-Winkelbereich,
Figur 3 einen Ablaufplan für einen möglichen Ablauf eines erfindungsgemäßen Verfahrens,
Figur 4 ein Beispiel für ein erfindungsgemäßes Verfahren zur Aufnahme von zwei Untersuchungsbereichen.

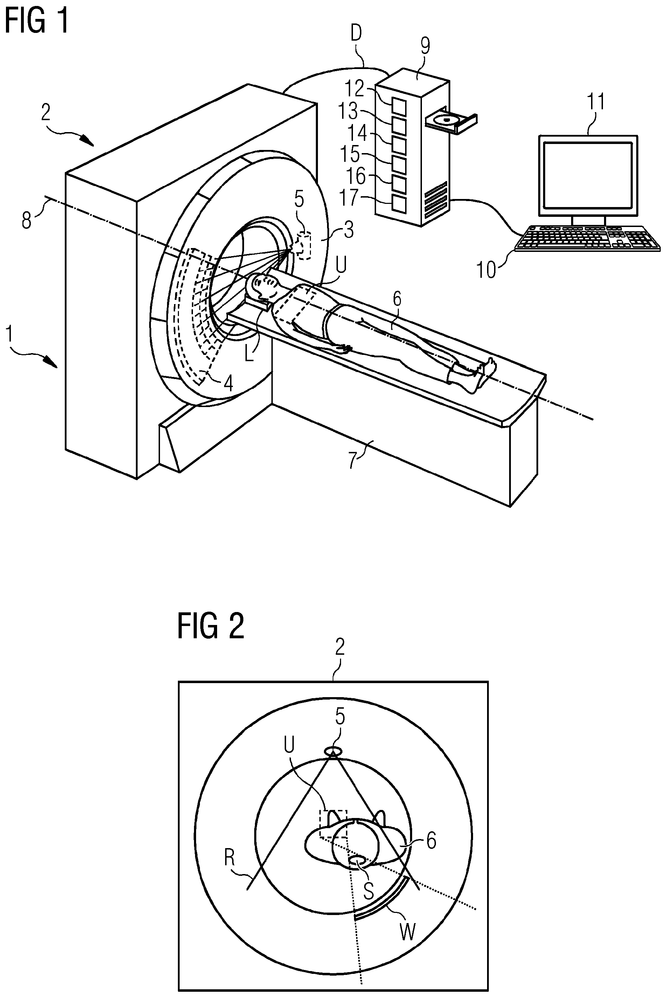

Figur 1 zeigt eine Ausführungsform eines Computertomographie-systems (CT-Systems) 1 mit einem Strahlungsdetektor 4 und einer Strahlungsquelle 5. Die Strahlungsquelle 5 ist dazu ausgebildet, den Strahlungsdetektor 4 mit Strahlung zu belichten. Das gezeigte CT-System 1 umfasst eine Gantry 2 mit einem Rotor 3. Der Rotor 3 umfasst als Strahlungsquelle 5 eine Röntgenquelle 5 und den Strahlungsdetektor 4, welcher ausgebildet ist Röntgenstrahlung zu detektieren.

Der Rotor 3 ist um die Rotationsachse 8 drehbar. Der Patient 6, ist auf der Patientenliege 7 gelagert und ist entlang der Rotationsachse 8 durch die Gantry 2 bewegbar. Der Kopf des Patienten 6 ist auf eine Lagerungshilfe L gebettet. Zur Steuerung des Bildgebungssystems 1 und/oder zur Erzeugung eines Bilddatensatzes basierend auf vom Strahlungsdetektor 4 detektierten Signalen ist die Recheneinheit 9 vorgesehen.

Es wird üblicherweise aus einer Vielzahl an Winkelrichtungen ein (Roh-) Röntgenbilddatensatz des Untersuchungsobjekts 6 mittels des Strahlungsdetektors 4 bei jeweils einer Strahlenergie aufgenommen, also zwei oder mehr Rohdatensätze. Anschließend kann basierend auf dem (Roh-) Röntgenbilddatensatz mittels eines mathematischen Verfahrens, beispielsweise umfassend eine gefilterte Rückprojektion oder ein iteratives Rekonstruktionsverfahren, ein (finaler) Bilddatensatz rekonstruiert werden.

Die Recheneinheit 9 dient hier als Steuereinrichtung 9 zur Steuerung des CT-Systems 1. Mit dieser Recheneinheit 9 ist eine Eingabeeinrichtung 10 und eine Ausgabeeinrichtung 11 verbunden. Die Eingabeeinrichtung 10 und die Ausgabeeinrichtung 11 können z.B. eine Interaktion durch einen Anwender oder die Darstellung eines erzeugten Bilddatensatzes B ermöglichen.

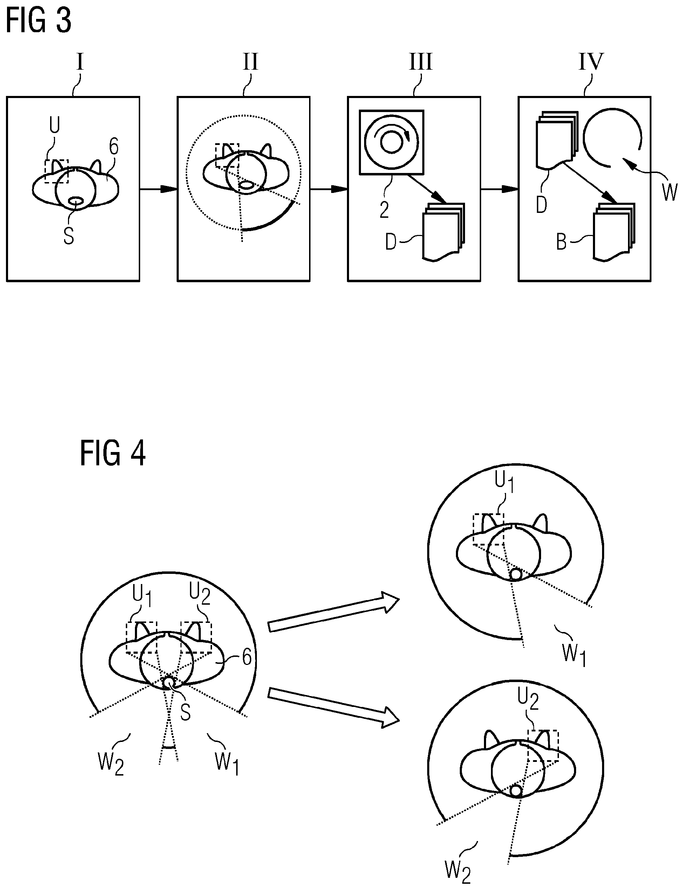

Die Steuereinrichtung 9 umfasst hier eine Datenschnittstelle 12 zum Empfang von CT-Daten, zur Ausgabe von Steuerbefehlen und zur Ausgabe von rekonstruierten Bilddatensätzen (s. dazu auch Figur 3).

Die Steuereinrichtung umfasst neben der Datenschnittstelle 12 noch eine Untersuchungseinheit 13, eine Störstruktur-Einheit 14, eine Berechnungseinheit 15, eine Rekonstruktionseinheit 16 und in diesem Beispiel auch eine Positionseinheit 17.

Die Untersuchungseinheit 13 dient zum Festlegen eines Untersuchungsbereichs U eines Patienten 6. Hier ist ein Bereich dargestellt, der für eine Untersuchung der Brust oder der Lunge geeignet sein könnte. In einem einfachen Fall kann der Untersuchungsbereich durch eine manuelle Eingabe festgelegt werden. Die Untersuchungseinheit 13 kann aber auch Werkzeuge zum Begrenzen des Untersuchungsbereichs U in Bildern oder zum festlegen des Untersuchungsbereichs U aus Vorgaben zur Untersuchung festlegen.

Die Störstruktur-Einheit 14 legt eine Anzahl von Störstrukturen S fest, wobei eine Störstruktur S eine Struktur ist, die innerhalb des Strahlengangs des Computertomographie-Systems 1 liegt und Röntgenstrahlung mit einer Stärke außerhalb eines Toleranzbereichs reflektiert oder absorbiert. Es kann z.B. vorgegeben sein, welche Strukturen im menschlichen Körper als Störstrukturen S anzusehen sind (z.B. die Wirbelsäule), dann kann ermittelt werden, welche dieser Störstrukturen S eine Messung des Untersuchungsbereichs U stören könnten. Diese werden als die (relevanten) Störstrukturen S festgelegt.

Die Berechnungseinheit 15 dient zum Berechnen der Strahlengänge der Röntgenstrahlen R einer CT-Untersuchung des Untersuchungsbereichs U und zum Ermitteln von Stör-Winkelbereichen W einer Gantry 2 des Computertomographie-Systems, bei denen Strahlengänge durch eine Störstruktur S und den Untersuchungsbereich U führen. Mit den bekannten Positionen der Röntgenquelle 5, ihrem bekannten Strahlengang und der bekannten Position einer festgelegten Störstruktur kann solch eine Berechnung einfach geometrisch durchgeführt werden.

Wie oben gesagt, dient die Datenschnittstelle 12 zur Ausgabe von Steuerbefehlen zum Durchführen einer CT-Aufnahme während der Rotator 3 der Gantry 2 rotiert, und zum Empfang von CT-Daten D.

Mit der Rekonstruktionseinheit 16 kann ein Bilddatensatz B aus den CT-Daten D unter Auslassung von ermittelten Stör-Winkelbereichen W rekonstruiert werden.

Die Datenschnittstelle 12 dient danach zum Ausgeben des rekonstruierten Bilddatensatzes B.

In diesem Beispiel umfasst die Steuereinrichtung 9 zusätzlich eine Positionseinheit 17 ausgelegt zum Ermitteln einer Position einer Lagerungshilfe L. Die Positionseinheit 17 ist dabei insbesondere dazu ausgelegt, basierend auf Bildern die Position abzuschätzen und/oder Informationen der Lagerungshilfe L zu empfangen und auszuwerten.

Figur 2 zeigt eine Störstruktur S, hier die Wirbelsäule, und einen Stör-Winkelbereich W. Es kann angenommen werden, dass ein Betrachter entlang der Drehachse der Figur 1 auf den Kopf des Patienten 6 blickt. Der Patient 6 ist hier etwas exzentrisch in der Gantry 2 positioniert, so dass der Untersuchungsbereich U um seine linke Brust im Zentrum liegt. Die (eigentlich unsichtbare) Wirbelsäule ist hier als Störstruktur S angedeutet. Die Röntgenquelle 5 mit ihrem kegelförmigen Röntgenstrahl R ist über dem Patienten 6 gezeigt. Bei einer Aufnahme rotiert sie um den Patienten 6 herum. Wenn sie sich im Stör-Winkelbereich W befindet, dann gehen Röntgenstrahlen R zuerst durch die Wirbelsäule und dann durch den Untersuchungsbereich U. Dies ist unerwünscht und daher wird der Stör-Winkelbereich W von der Aufnahme (oder zumindest von der Rekonstruktion) ausgenommen.

Figur 3 zeigt einen Ablaufplan für einen möglichen Ablauf eines erfindungsgemäßen Verfahrens zur Steuerung eines Computertomographie-Systems 1 wie es z.B. in Figur 1 dargestellt ist.

In Schritt I werden ein Untersuchungsbereich U eines Patienten 6 und eine Störstruktur S festgelegt. Diese Störstruktur ist in diesem Beispiel die Wirbelsäule wie in Figur 2 gezeigt.

In Schritt II wird durch Berechnen der Strahlengänge der Röntgenstrahlen R, die durch eine Störstruktur S und den Untersuchungsbereich U führen, ein Stör-Winkelbereich W einer Gantry 2 des Computertomographie-Systems 1 ermittelt.

In Schritt III wird eine CT-Aufnahme durchgeführt, während die Gantry 2 rotiert (d.h. ihr Rotator 3 rotiert). Dabei werden CT-Daten D aufgenommen. Es kann davon ausgegangen werden, dass zur Verringerung der Dosis die Röntgenquelle 5 im Stör-Winkelbereich W ausgeschaltet wird.

In Schritt IV wird ein Bilddatensatz B aus den CT-Daten D unter Auslassung von ermittelten Stör-Winkelbereichen W rekonstruiert und danach ausgegeben.

Figur 4 zeigt ein Beispiel für ein erfindungsgemäßes Verfahren zur Aufnahme. Es werden dabei an einem Patienten 6 zwei Untersuchungsbereiche U₁, U₂ festgelegt und Stör-Winkelbereiche W₁, W₂ für beide Untersuchungsbereiche U₁, U₂ separat ermittelt. Ein Stör-Winkelbereich W₁, W₂ wird dabei einem Untersuchungsbereich U₁, U₂ zugeordnet, wenn Strahlengänge durch eine Störstruktur S und den betreffenden Untersuchungsbereich U₁, U₂ führen. Für jeden Untersuchungsbereich U₁, U₂ wird dann ein Bilddatensatz B separat unter Auslassung von ermittelten Stör-Winkelbereichen W₁, W₂ des betreffenden Untersuchungsbereichs U₁, U₂ rekonstruiert.

In diesem Falle überlappen die Stör-Winkelbereiche W₁, W₂ nicht. Das bedeutet, dass bei der Aufnahme der CT-Daten die Röntgenquelle nicht ausgeschaltet wird, da die Daten von 360° der Rekonstruktion zumindest einem der Untersuchungsbereiche U₁, U₂ dienen können.

Gäbe es einen Stör-Winkelbereich W der beiden Untersuchungsbereichen U₁, U₂ zugeordnet wäre (ein identischer Stör-Winkelbereiche W), dann könnte die zur Aufnahme verwendete Röntgenquelle 5 in diesem Stör-Winkelbereich W abgeschaltet werden.

Es wird abschließend noch einmal darauf hingewiesen, dass es sich bei den vorhergehend detailliert beschriebenen Figuren lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Gerät" nicht aus, dass die betreffenden Komponenten aus mehreren zusammenwirkenden Teil-Komponenten bestehen können, die gegebenenfalls auch räumlich verteilt sein können. Der Ausdruck "eine Anzahl" ist als "mindestens eins" zu verstehen. Unabhängig vom grammatikalischen Geschlecht eines bestimmten Begriffes sind Personen mit männlicher, weiblicher oder anderer Geschlechteridentität mit umfasst.

## Patentansprüche

1. Verfahren zur Steuerung eines Computertomographie-Systems (1) umfassend die Schritte:
- Festlegen eines Untersuchungsbereichs (U) eines Untersuchungsobjekts (6),
- Festlegen einer Anzahl von Störstrukturen (S), wobei eine Störstruktur (S) eine Struktur ist, die innerhalb des Strahlengangs des Computertomographie-Systems (1) liegt und Röntgenstrahlung mit einer Stärke außerhalb eines Toleranzbereichs reflektiert oder absorbiert,
- Berechnen der Strahlengänge der Röntgenstrahlen (R) einer CT-Untersuchung des Untersuchungsbereichs (U) und Ermitteln von Stör-Winkelbereichen (W) einer Gantry (2) des Computertomographie-Systems, bei denen Strahlengänge durch eine Störstruktur (S) und den Untersuchungsbereich (U) führen,
- Durchführen einer CT-Aufnahme während die Gantry (2) rotiert, und Aufnehmen von CT-Daten (D),
- Rekonstruieren eines Bilddatensatzes (B) aus den CT-Daten (D) unter Auslassung von ermittelten Stör-Winkelbereichen (W),
- Ausgeben des Bilddatensatzes (B).

2. Verfahren nach Anspruch 1, wobei die CT-Daten (D) unter Auslassung von ermittelten Stör-Winkelbereichen (W) aufgenommen werden, bevorzugt wobei eine zur Aufnahme verwendete Röntgenquelle (5) in Stör-Winkelbereichen (W) abgeschaltet wird oder zumindest ihre Strahlungsleistung verringert wird, bevorzugt wobei Strahlparameter, insbesondere die Beschleunigungsspannung und/oder die Stromstärke der Röntgenquelle (5), für die gesamte Aufnahme basierend auf einem gesamten Winkelbereich von 360° abzüglich der Stör-Winkelbereiche (W) bestimmt werden.

3. Verfahren nach einem der vorangehenden Ansprüche, wobei als Stör-Winkelbereich (W) ein Winkelbereich angesehen wird, bei dem Strahlen einer Röntgenquelle (5) der Gantry (2) zuerst durch eine Störstruktur (S) und danach durch den Untersuchungsbereich (U) führen.

4. Verfahren nach einem der vorangehenden Ansprüche, wobei das Festlegen einer Störstruktur (S) auf einem Topogramm des Untersuchungsobjekts (6), einer Kameraaufnahme des Untersuchungsobjekts (6) oder einem Modell des Untersuchungsobjekts (6) basiert, bevorzugt wobei das Untersuchungsobjekt (6) ein menschlicher oder tierischer Körper ist und die Störstruktur (S) ein Knochen, eine Lagerungshilfe (L) oder ein Teil der Gantry (2) ist.

5. Verfahren nach einem der vorangehenden Ansprüche, wobei das Untersuchungsobjekt (6) mittels einer Lagerungshilfe (L) gelagert oder mit einer Fixation fixiert wird und das Festlegen einer Störstruktur (S) auf einer Position und einer Form der Lagerungshilfe (L) oder Fixation basiert,
bevorzugt wobei das Isozentrum des Untersuchungsbereichs (U) nach Lagerung des Untersuchungsobjekts (6) mittels der Lagerungshilfe (L) festgelegt wird, insbesondere automatisiert mittels einer Bilderkennung oder mittels einer Kommunikation zwischen Lagerungshilfe (L) und Computertomographie-System (1).

6. Verfahren nach einem der vorangehenden Ansprüche, wobei im Rahmen der Ermittlung der Stör-Winkelbereiche (W) überprüft wird, ob unter Berücksichtigung des gesamten Winkelbereichs von 360° abzüglich der Stör-Winkelbereiche (W) der verbleibende Winkelbereich überlagert mit dem verbleibenden Winkelbereich gedreht um 180° einen durchgehenden Winkelbereich von 360° aufweist,
bevorzugt wobei in dem Fall, dass der verbleibende Winkelbereich kleiner als 360° ist, ein fehlender Winkelbereich vor der Rekonstruktion des Bilddatensatzes (B) den CT-Daten (D) simulierte Daten hinzugefügt werden, die Aufnahmen im fehlenden Winkelbereich simulieren und/oder ein fehlender Winkelbereich aus er Anzahl der Stör-Winkelbereiche (W) entfernt wird, wobei insbesondere ein Grad der Störung im fehlenden Winkelbereich und einem dazu 180° gedrehten Winkelbereich ermittelt wird und derjenige Winkelbereich aus den Stör-Winkelbereichen (W) entfernt wird, der den geringeren Grad der Störung aufweist.

7. Verfahren nach einem der vorangehenden Ansprüche, wobei zwei oder mehr Untersuchungsbereiche (U1, U2) des Untersuchungsobjekts (6) festgelegt werden, Stör-Winkelbereiche (W1, W2) für beide Untersuchungsbereiche (U1, U2) separat ermittelt werden, wobei ein Stör-Winkelbereich (W1, W2) einem Untersuchungsbereich (U1, U2) zugeordnet wird, wenn Strahlengänge durch eine Störstruktur (S) und den betreffenden Untersuchungsbereich (U1, U2) führen, und für jeden Untersuchungsbereich (U1, U2) ein Bilddatensatz (B) separat unter Auslassung von ermittelten Stör-Winkelbereichen (W1, W2) des betreffenden Untersuchungsbereichs (U1, U2) rekonstruiert wird, bevorzugt wobei ermittelt wird, welche den Untersuchungsbereichen (U1, U2) zugeordneten Stör-Winkelbereiche (W) identisch sind, die CT-Daten (D) unter Auslassung der identischen Stör-Winkelbereiche (W) aufgenommen werden, bevorzugt wobei eine zur Aufnahme verwendete Röntgenquelle (5) in den identischen Stör-Winkelbereichen (W) abgeschaltet wird oder zumindest ihre Strahlungsleistung verringert wird.

8. Verfahren nach einem der vorangehenden Ansprüche, wobei bei der Rekonstruktion eines Bilddatensatzes (B) eine Kombination einer Data-sparsity-Rekonstruktionstechnik mit einem iterativen Lösungsansatz verwendet wird, bevorzugt eine Zumischung von Projektionen zur Darstellung von Strukturen.

9. Verfahren nach einem der vorangehenden Ansprüche, wobei der Untersuchungsbereich (U) die Brust oder die Lunge ist und wobei eine bevorzugte Störstruktur (S) die Wirbelsäule ist.

10. Steuereinrichtung (9) zur Steuerung eines Computertomographie-Systems (1) umfassend:
- eine Untersuchungseinheit (13) ausgelegt zum Festlegen eines Untersuchungsbereichs (U) eines Untersuchungsobjekts (6),
- eine Störstruktur-Einheit (14) ausgelegt zum Festlegen einer Anzahl von Störstrukturen (S), wobei eine Störstruktur (S) eine Struktur ist, die innerhalb des Strahlengangs des Computertomographie-Systems (1) liegt und Röntgenstrahlung mit einer Stärke außerhalb eines Toleranzbereichs reflektiert oder absorbiert,
- eine Berechnungseinheit (15) ausgelegt zum Berechnen der Strahlengänge der Röntgenstrahlen (R) einer CT-Untersuchung des Untersuchungsbereichs (U) und Ermitteln von Stör-Winkelbereichen (W) einer Gantry (2) des Computertomographie-Systems, bei denen Strahlengänge durch eine Störstruktur (S) und den Untersuchungsbereich (U) führen,
- eine Datenschnittstelle (12) ausgelegt zur Ausgabe von Steuerbefehlen zum Durchführen einer CT-Aufnahme während die Gantry (2) rotiert, und zum Empfang von CT-Daten (D),
- eine Rekonstruktionseinheit (16) ausgelegt zum Rekonstruieren eines Bilddatensatzes (B) aus den CT-Daten (D) unter Auslassung von ermittelten Stör-Winkelbereichen (W),
- eine Datenschnittstelle (12) ausgelegt zum Ausgeben des Bilddatensatzes (B).

11. Steuereinrichtung nach Anspruch 10, umfassend eine Positionseinheit (17) ausgelegt zum Ermitteln einer Position einer Lagerungshilfe (L) oder eine Fixation,
bevorzugt wobei die Positionseinheit (17) dazu ausgelegt ist, basierend auf Bildern die Position abzuschätzen und/oder Informationen der Lagerungshilfe (L) zu empfangen und auszuwerten.

12. Computertomographie-System (1) umfassend eine Steuereinrichtung (9) nach einem der Ansprüche 10 bis 11 und/oder ausgelegt zur Durchführung eines Verfahrens nach einem der Ansprüche 1 bis 9.

13. Computerprogrammprodukt, umfassend Befehle, die bei der Ausführung des Programms durch einen Computer diesen veranlassen, die Schritte des Verfahrens nach einem der Ansprüche 1 bis 9 auszuführen, wobei die Durchführung einer CT-Aufnahme der Übersendung von entsprechenden Steuerbefehlen und dem Empfang von CT-Daten (D) entspricht.

14. Computerlesbares Speichermedium, umfassend Befehle, die bei der Ausführung durch einen Computer diesen veranlassen, die Schritte des Verfahrens nach einem der Ansprüche 1 bis 9 auszuführen, wobei die Durchführung einer CT-Aufnahme der Übersendung von entsprechenden Steuerbefehlen und dem Empfang von CT-Daten (D) entspricht.

## Claims

1. Method for controlling a computed tomography system (1), comprising the following steps:
- defining an examination region (U) of an examination object (6),
- defining a number of interfering structures (S), wherein an interfering structure (S) is a structure which is located within the beam path of the computed tomography system (1) and reflects or absorbs X-ray radiation with a strength outside of a tolerance range,
- calculating the beam paths of the X-rays (R) of a CT examination of the examination region (U) and ascertaining interference angle ranges (W) of a gantry (2) of the computed tomography system at which beam paths run through an interfering structure (S) and the examination region (U),
- carrying out a CT scan while the gantry (2) rotates, and recording CT data (D),
- reconstructing an image dataset (B) from the CT data (D) with the omission of ascertained interference angle ranges (W),
- outputting the image dataset (B).

2. Method according to claim 1, wherein the CT data (D) is recorded with the omission of ascertained interference angle ranges (W), preferably wherein an X-ray source (5) used for recording is switched off in interference angle ranges (W) or at least its radiation power is reduced, preferably wherein beam parameters, in particular the acceleration voltage and/or the current strength of the X-ray source (5), are determined for the entire recording on the basis of an entire angle range of 360° minus the interference angle ranges (W).

3. Method according to one of the preceding claims, wherein an angle range at which beams of an X-ray source (5) of the gantry (2) run firstly through an interfering structure (S) and then through the examination region (U) is regarded as an interference angle range (W).

4. Method according to one of the preceding claims, wherein defining an interfering structure (S) is based on a topogram of the examination object (6), a camera recording of the examination object (6) or a model of the examination object (6), preferably wherein the examination object (6) is a human or animal body and the interfering structure (S) is a bone, a positioning aid (L) or a part of the gantry (2).

5. Method according to one of the preceding claims, wherein the examination object (6) is positioned by means of a positioning aid (L) or fixed with a fixation and defining an interfering structure (S) is based on a position and a form of the positioning aid (L) or fixation,
preferably wherein the isocentre of the examination region (U) is defined after positioning of the examination object (6) by means of the positioning aid (L), in particular automatically by means of an image recognition or by means of a communication between positioning aid (L) and computed tomography system (1).

6. Method according to one of the preceding claims, wherein in the course of ascertaining the interference angle ranges (W) it is checked whether considering the entire angle range of 360° minus the interference angle ranges (W), the remaining angle range overlaid with the remaining angle range rotated about 180° has a continuous angle range of 360°,
preferably wherein in the case where the remaining angle range is less than 360°, a missing angle range, simulated data, which simulates recordings in the missing angle range, is added to the CT data (D) before reconstruction of the image dataset (B) and/or a missing angle range is removed from the number of interference angle ranges (W), wherein, in particular, a degree of interference in the missing angle range and an angle range rotated 180° thereto is ascertained and that angle range which has the smaller degree of interference is removed from the interference angle ranges (W) .

7. Method according to one of the preceding claims, wherein two or more examination regions (U1, U2) of the examination object (6) are defined, interference angle ranges (W1, W2) are separately ascertained for the two examination regions (U1, U2), wherein an interference angle range (W1, W2) is associated with an examination region (U1, U2) if beam paths run through an interfering structure (S) and the relevant examination region (U1, U2), and for each examination region (U1, U2) an image dataset (B) is separately reconstructed with the omission of ascertained interference angle ranges (W1, W2) of the relevant examination region (U1, U2),
preferably wherein it is ascertained which interference angle ranges (W) associated with the examination regions (U1, U2) are identical, the CT data (D) is recorded with the omission of the identical interference angle ranges (W), preferably wherein an X-ray source (5) used for recording is switched off in the identical interference angle ranges (W) or at least its radiant power is reduced.

8. Method according to one of the preceding claims, wherein during reconstruction of an image dataset (B) a combination of a data sparsity reconstruction technique with an iterative solution approach is used, preferably an addition of projections in order to represent structures.

9. Method according to one of the preceding claims, wherein the examination region (U) is the chest or the lungs and wherein a preferred interfering structure (S) is the spinal column.

10. Control facility (9) for controlling a computed tomography system (1), comprising:
- an examination unit (13) designed for defining an examination region (U) of an examination object (6),
- an interfering structure unit (14) designed for defining a number of interfering structures (S), wherein an interfering structure (S) is a structure, which is located within the beam path of the computed tomography system (1) and reflects or absorbs X-ray radiation with a strength outside of a tolerance range,
- a calculation unit (15) designed for calculating the beam paths of the X-rays (R) of a CT examination of the examination region (U) and ascertaining interference angle ranges (W) of a gantry (2) of the computed tomography system at which beam paths run through an interfering structure (S) and the examination region (U),
- a data interface (12) designed for outputting control commands for carrying out a CT scan while the gantry (2) rotates, and for receiving CT data (D),
- a reconstruction unit (16) designed for reconstructing an image dataset (B) from the CT data (D) with the omission of ascertained interference angle ranges (W),
- a data interface (12) designed for outputting the image dataset (B).

11. Control facility according to claim 10, comprising a position unit (17) designed for ascertaining a position of a positioning aid (L) or a fixation,
preferably wherein the position unit (17) is designed to estimate the position on the basis of images and/or receive and evaluate items of information of the positioning aid (L).

12. Computed tomography system (1), comprising a control facility (9) according to one of claims 10 to 11 and/or designed for carrying out a method according to one of claims 1 to 9.

13. Computer program product, comprising commands which on execution of the program by a computer prompt it to execute the steps of the method according to one of claims 1 to 9, wherein carrying out a CT recording corresponds to the transmission of corresponding control commands and the receiving of CT data (D).

14. Computer-readable storage medium, comprising commands which on execution by a computer prompt it to execute the steps of the method according to one of claims 1 to 9, wherein carrying out a CT recording corresponds to the transmission of corresponding control commands and the receiving of CT data (D) .

## Revendications

1. Procédé de commande d'un système (1) de tomodensitométrie assisté par ordinateur comprenant les stades :
- détermination d'une partie (U) à examiner d'un objet (6) à examiner,
- détermination d'un nombre de structures (S) perturbatrices, dans lequel une structure (S) perturbatrice est une structure, qui se trouve dans le trajet de rayonnement du système (1) de tomodensitométrie assisté par ordinateur et qui réfléchit ou absorbe du rayonnement X en une intensité en dehors d'une plage de tolérance,
- calcul des trajets de rayonnements X (R) d'un examen CT de la partie (U) à examiner et détermination de plages (W) angulaires de perturbation d'un portique (2) du système de tomodensitométrie assisté par ordinateur, dans lequel des trajets de rayonnements passent dans une structure (S) perturbatrice et dans la partie (U) à examiner,
- exécution d'un enregistrement CT pendant que le portique (2) tourne et enregistrement de données CT (D),
- reconstruction d'un ensemble (B) de données d'image à partir des données CT (D) en supprimant des plages (W) angulaires de perturbation déterminées,
- sortie de l'ensemble (B) de données d'image.

2. Procédé suivant la revendication 1, dans lequel on enregistre les données CT (D) en supprimant des plages (W) angulaires de perturbation déterminées, dans lequel de préférence on met hors circuit dans des plages (W) angulaires de perturbation une source (5) de rayons X utilisée pour l'enregistrement ou du moins on diminue sa puissance de rayonnement, dans lequel de préférence on détermine des paramètres de rayonnement, en particulier la tension d'accélération et/ou l'intensité du courant de la source (5) de rayons X, pour l'enregistrement d'ensemble sur la base d'une plage angulaire d'ensemble de 360° avec déduction des plages (W) angulaires de perturbation.

3. Procédé suivant l'une des revendications précédentes, dans lequel on considère comme plage (W) angulaire de perturbation, une plage angulaire, dans laquelle le rayonnement d'une source (5) de rayons X du portique (2) passe d'abord dans une structure (S) perturbatrice et ensuite dans la partie (U) à examiner.

4. Procédé suivant l'une des revendications précédentes, dans lequel la détermination d'une structure (S) perturbatrice se fonde sur un topogramme de l'objet (6) à examiner, d'un enregistrement par appareil photographique de l'objet (6) à examiner ou d'un modèle de l'objet (6) à examiner, dans lequel de préférence l'objet (6) à examiner est un corps humain ou animal et la structure (S) perturbatrice est un os, une aide (L) à la mise en position ou une partie du portique (2).

5. Procédé suivant l'une des revendications précédentes, dans lequel on met l'objet (6) à examiner en position au moyen d'une aide (L) à la mise en position ou on le fixe au moyen d'une fixation et la détermination d'une structure (S) perturbatrice repose sur une position et une forme de l'aide (L) à la mise en position ou de la fixation,
dans lequel de préférence on détermine l'isocentre de la partie (U) à examiner après le positionnement de l'objet (6) à examiner au moyen de l'aide (L) à la mise en position, en particulier d'une manière automatisée au moyen d'une reconnaissance d'images ou au moyen d'une communication entre l'aide (L) à la mise en position et le système (1) de tomodensitométrie assisté par ordinateur.

6. Procédé suivant l'une des revendications précédentes, dans lequel, dans le cadre de la détermination des plages (W) angulaires de perturbation, on contrôle si, en tenant compte de toute la plage angulaire de 360° diminuée des plages (W) angulaires de perturbation, la plage angulaire restante, superposée à la plage angulaire restante tournée de 180°, a une plage angulaire continue de 360°,
dans lequel, dans le cas où la plage angulaire restante est plus petite que 360°, on ajoute une plage angulaire manquante, avant la reconstruction de l'ensemble (B) de données d'image, à des données simulant les données CT (D), qui simulent les enregistrements dans la plage angulaire manquante et/ou on élimine une plage angulaire manquante à l'exception d'un nombre des plages (W) angulaires de perturbation, dans lequel on détermine, en particulier, un degré de la perturbation dans la plage angulaire manquante et une plage angulaire tournée de 180° par rapport à celle-ci et on élimine la plage angulaire des plages (W) angulaires de perturbation, qui a le degré de perturbation le plus petit.

7. Procédé suivant l'une des revendications précédentes, dans lequel on détermine deux ou plusieurs parties (U1, U2) à examiner de l'objet (6) à examiner, on détermine séparément des plages (W1, W2) angulaires de perturbation pour les deux parties (U1, U2) à examiner, dans lequel on affecte une plage (W1, W2) angulaire de perturbation à une partie (U1, U2) à examiner, si des trajets de rayonnements passent par une structure (S) de perturbation et par la partie (U1, U2) à examiner concernée et, pour chaque partie (U1, U2) à examiner, on reconstruit un ensemble (B) de données d'image séparément en supprimant des plages (W1, W2) angulaires de perturbation déterminées de la partie (U1, U2) à examiner concernée, dans lequel on détermine de préférence les plages (W) angulaires de perturbation affectées aux parties (U1, U2) à examiner qui sont identiques, on enregistre les données CT (D) en supprimant les plages (W) angulaires de perturbation identiques, dans lequel, de préférence, on met hors-circuit, dans les plages (W) angulaires de perturbation identiques, une source (5) de rayons X utilisée pour l'enregistrement ou du moins on en diminue la puissance de rayonnement.

8. Procédé suivant l'une des revendications précédentes, dans lequel, lors de la reconstruction d'un ensemble (B) de données d'image, on utilise une technique de reconstruction data-sparsity, comprenant une équation de résolution itérative, de préférence un mélange de projections pour la représentation de structures.

9. Procédé suivant l'une des revendications précédentes, dans lequel la partie (U) à examiner est le buste ou le poumon et dans lequel une structure (S) de perturbation préférée est la colonne vertébrale.

10. Dispositif (9) de commande pour la commande d'un système (1) de tomodensitométrie assisté par ordinateur comprenant :
- une unité (13) d'examen conçue pour déterminer une partie (U) à examiner d'un objet (6) à examiner,
- une unité (14) de structure de perturbation conçue pour déterminer un nombre de structures (S) de perturbation, dans lequel une structure (S) de perturbation est une structure, qui se trouve dans le trajet du rayonnement du système (1) de tomodensitométrie assisté par ordinateur et qui réfléchit ou absorbe du rayonnement X en une intensité en dehors d'une plage de tolérance,
- une unité (15) de calcul conçue pour le calcul des trajets du rayonnement X (R) d'un examen CT de la partie (U) à examiner et pour la détermination de plages (W) angulaires de perturbation d'un portique (2) du système de tomodensitométrie assisté par ordinateur, dans lesquelles des trajets de rayonnements passent par une structure (S) de perturbation et par la partie (U) à examiner,
- une interface (12) de données conçue pour l'émission d'instructions de commande pour effectuer un enregistrement CT pendant que le portique (2) tourne et pour recevoir des données CT (D),
- une unité (16) de reconstruction conçue pour la reconstruction d'un ensemble (B) de données d'image à partir des données CT (D) en supprimant des plages (W) angulaires de perturbation déterminées,
- une interface (12) de données conçue pour donner l'ensemble (B) de données d'image.

11. Dispositif de commande suivant la revendication 10, comprenant une unité (17) de mise en position conçue pour déterminer une position d'une aide (L) à la mise en position ou une fixation,
dans lequel de préférence l'unité (17) de mise en position est conçue pour évaluer la position sur la base d'images et/ou pour recevoir des informations de l'aide (L) à la mise en position et les évaluer.

12. Système (1) de tomodensitométrie assisté par ordinateur comprenant une unité (9) de commande suivant l'une des revendications 10 à 11 et/ou conçue pour effectuer un procédé suivant l'une des revendications 1 à 9.

13. Produit de programme d'ordinateur, comprenant des instructions, qui, lors de l'exécution du programme par ordinateur, font que celles-ci exécutent les stades du procédé suivant l'une des revendications 1 à 9, dans lequel l'exécution d'un enregistrement CT correspond à l'envoi d'instructions de commande correspondantes et à la réception de données CT (D).

14. Support de mémoire déchiffrable par ordinateur, comprenant des instructions, qui, lors de l'exécution par ordinateur, font que celles-ci exécutent les stades du procédé suivant l'une des revendications 1 à 9, l'exécution d'un enregistrement CT correspondant à l'envoi d'instructions de commande correspondantes et à la réception de données CT (D).
